# EUROPÄISCHE PATENTANMELDUNG

(11) **EP 0 893 120 A2**
(43) Veröffentlichungstag der Anmeldung: **27.01.1999**
(21) Anmeldenummer: 98113304.4
(22) Anmeldetag: 16.07.1998
(51) Int. Cl.: A61K 7/50

(54) **Selbstemulgierende Zubereitungen**

(30) Priorität: 25.07.1997 DE 19732015
(71) Anmelder: Henkel Kommanditgesellschaft auf Aktien, 40589 Düsseldorf-Holthausen (DE)
(72) Erfinder: Bigorra, Joaquin, Dr., 08203 Sabadell (ES); Prat Queralt, Ester, Dr., 08238 Alella (ES); Pi Subirana, Rafael, Dr., 08400 Granollers (ES)

(57) **Zusammenfassung**

Vorgeschlagen werden neue selbstemulgierende Zubereitungen, enthaltend
(a) 20 bis 25 Gew.-% Esterquats
(b) 60 bis 65 Gew.-% Ölkörper und
(c) 10 bis 20 Gew.-% Emulgatoren vom Typ der Partialglyceride von Fettsäuren mit 6 bis 14 Kohlenstoffatomen,
mit der Maßgabe, daß sich die Mengenangaben zu 100 Gew.-% ergänzen. Die Mischungen bilden in Wasser spontan Emulsionen und können unmittelbar beispielsweise als Haarkonditioniermittel verwendet werden.

## Beschreibung

### Gebiet der Erfindung

Die Erfindung betrifft selbstemulgierende Zubereitungen mit einem definierten Gehalt an Esterquats, Ölkörpern und ausgewählten Emulgatoren sowie die Verwendung der Zubereitungen zur Herstellung von kosmetischen Mitteln.

### Stand der Technik

Moderne Haarbehandlungsmittel enthalten neben oberflächenaktiven Substanzen, die für die Reinigung der Haare zuständig sind, vor allem auch kationische Verbindungen, die auf die Fasern aufziehen und ihnen gleichzeitig einen angenehmen Weichgriff verleihen und die elektrostatische Aufladung herabsetzen. Typische Beispiele für solche Kationtenside sind quaternierte Fettsäurealkanolaminester, die üblicherweise als Esterquats bezeichnet werden. Zur Herstellung von Haarbehandlungsmitteln, speziell Haarkonditioniermitteln, werden die einzelnen Komponenten üblicherweise einzeln in Wasser gelöst oder dispergiert, wobei die Einsatzstoffe sowohl wasserfrei vorliegen können als auch als wäßrige und/oder alkoholische Lösungen. Üblicherweise erfolgt die Herstellung in einem Heißprozeß, d.h. die Vermischung findet bei 70 bis 80°C unter starkem Rühren statt. Ziel sind dabei möglichst homogene, feinteilige und damit auch lagerstabile Emulsionen, die insbesondere im Laufe der Zeit nicht ihre Viskosität verändern.

Aus der Europäischen Patentschritt **EP-B1 0689532** (Henkel) ist ein Verfahren zur Herstellung von schuppbaren Esterquats bekannt, bei dem man die Quaternierung der Triethanolaminfettsäureester in Gegenwart von Fettalkoholen oder Dialkylethern sowie gegebenenfalls zusätzlich Fettalkoholpolyglycolethern, ethoxylierten Partialglyceriden und/oder Alkyloligoglucosiden vornimmt. Die Quaternierung der Triethanolaminfettsäureester in Gegenwart von Alkyloligoglucosiden, Fettsäure-N-alkylglucamiden, ethoxylierten Partialglyceriden oder Oligoglycerinen wird in der Deutschen **Patentschrift DE-C1 4335782** (Henkel) beschrieben. Haarbehandlungsmittel, die Alkylglucoside zusammen mit Esterquats und Ölkörpern enthalten, sind ferner aus der Europäischen Patentschritt **EP-B1 0680314** (Henkel) bekannt.

Obschon Haarbehandlungsmittel in Form wäßriger Emulsionen mit Esterquats und Ölkörpern im Markt erhältlich sind, besteht doch nach wie vor ein Bedürfnis nach einem selbstemulgierenden Konzentrat, das in Wasser spontan eine homogene und stabile Emulsion bildet, die unmittelbar als kosmetisches Mittel eingesetzt werden kann. Die Aufgabe der vorliegenden Erfindung hat somit darin bestanden, selbstemulgierende Zubereitungen auf Basis von Esterquats zur Verfügung zu stellen, die beim Eintragen in Wasser spontan Emulsionen bilden, die sich durch besondere Homogenität und Lagerstabilität auszeichnen und unmittelbar als kosmetische Mittel verwendet werden können.

### Beschreibung der Erfindung

Gegenstand der Erfindung sind selbstemulgierende Zubereitungen, enthaltend
(a) 20 bis 25 Gew.-% Esterquats
(b) 60 bis 65 Gew.-% Ölkörper und
(c) 10 bis 20 Gew.-% Emulgatoren vom Typ der Partialglyceride von Fettsäuren mit 6 bis 14 Kohlenstoffatomen,
mit der Maßgabe, daß sich die Mengenangaben zu 100 Gew.-% ergänzen.

Überraschenderweise wurde gefunden, daß die Zubereitungen selbstemulgierend sind, d.h. beim Eintragen in Wasser spontan stabile und sehr homogene Emulsionen ergeben, die unmittelbar, beispielsweise als Haarkonditioniermittel eingesetzt werden können. Die Erfindung schließt die Erkenntnis ein, daß die Emulsionen schon unter Verwendung sehr geringer Mengen der Zubereitungen entstehen und auch bei Temperaturbelastung nicht nennenswert eindicken, d.h. eine konstante Viskosität besitzen.

### Esterquats

Unter der Bezeichnung 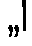 Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 43 08 794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens. Surf. Det., 30, 186 (1993),** M.Brock in **Tens. Surf. Det. 30, 394 (1993),** R.Lagerman et al. in **J. Am. Oil. Chem Soc., 71, 97 (1994)** sowie I.Shapiro in **Cosm. Toil. 109 77 (1994)** erschienen.

Die quaternierten Fettsäuretriethanolaminestersalze folgen der Formel **(I)**, 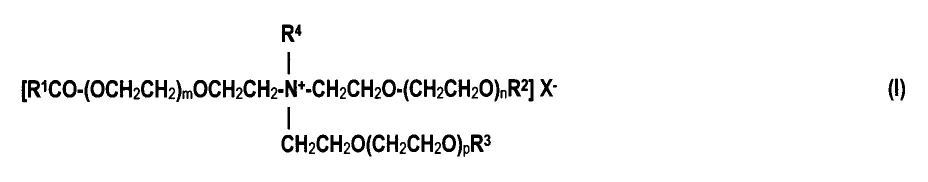 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsaure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsauren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Tag- bzw. Palmfettsäure (Iodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 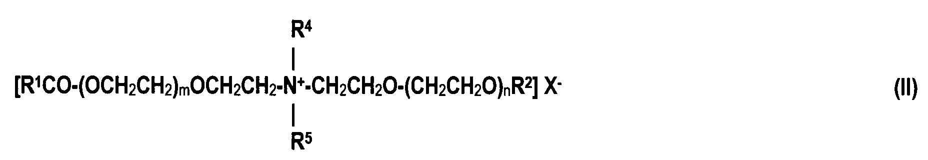 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 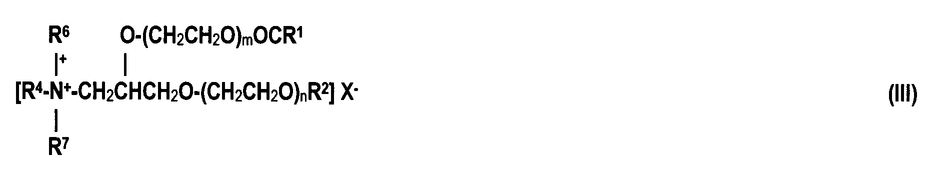 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können.

### Ölkörper

Die erfindungsgemäßen Zubereitungen enthalten als Ölkörper vorzugsweise Fettalkohole der Formel **(IV)**, 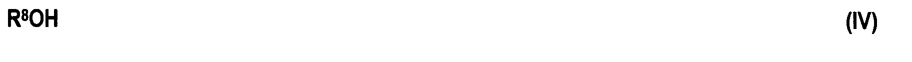 in der R⁸ für einen lienaren oder verzweigten Alkyl- und/oder Alkenylrest mit 6 bis 22 Kohlenstoffatomen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol.

Zubereitungen, die Fettalkohole als Ölkörper enthalten, weisen eine vergleichsweise hohe Viskosität auf. Sollten niedrigviskose Zubereitungen gewünscht werden, empfiehlt es sich, die als Ölkörper bevorzugten Fettalkohole ganz oder zu einem Teil, beispielsweise zu 25 bis 75 Gew.-% durch langkettige Partialglyceride auszutauschen, die der Formel **(V)** folgen, 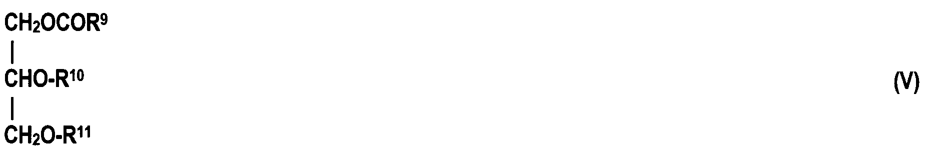 in der R⁹CO für einen linearen oder verzweigten, gesättigten und/oder ungesättigten Acylrest mit 16 bis 22 Kohlenstoffatomen, R¹⁰ und R¹¹ unabhängig voneinander für Wasserstoff oder R⁹CO steht, mit der Maßgabe, daß mindestens einer der beiden Reste R¹⁰ und R¹¹ Wasserstoff darstellt. Typische Beispiele sind technische Mono/Diglyceride auf Basis von Palmitinsäure, Stearinsäure, Isostearinsäure, Ölsäure, Elaidinsäure, Behensäure und/oder Erucasäure. Vorzugsweise wird Stearinsäuremonoglycerid eingesetzt.

Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

### Emulgatoren

Die erfindungsgemäßen Zubereitungen enthalten als Emulgatoren kurzkettige Partialglyceride, die vorzugsweise der Formel **(VI)** folgen, 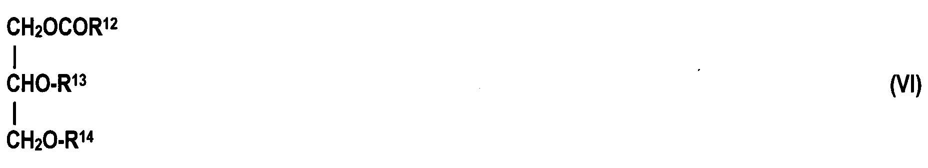 in der R¹²CO für einen Acylrest mit 12 bis 14 Kohlenstoffatomen, R¹³ und R¹⁴ unabhängig voneinander für Wasserstoff oder R¹²CO steht, mit der Maßgabe, daß mindestens einer der beiden Reste R¹³ und R¹⁴ Wasserstoff darstellt. Typische Beispiele hierfür sind die Mono- und/oder Diglyceride auf Basis von C₈-C₁₀-Vorlauffettsäure, Laurinsäure und Myristinsäure sowie deren technische Mischungen, insbesondere auch Mono- und/oder Diglyceride auf Basis entsprechender Kokosfettsäureschnitte. Vorzugsweise wird Laurinsäuremonoglycerid eingesetzt.

Als Co-Emulgatoren kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind hinreichend aus der Literatur bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche obenflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkyl-aminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Vorzugsweise enthalten die erfindungsgemäßen Zubereitungen als Co-Emulgtoren Alkyl- und/oder Alkenyloligoglykoside und Polyolpoly-12-hydrxoystearate und insbesondere Alkyloligoglucoside und/oder Polyglycerinpoly-12-hydrxoystearat.

### Herstellverfahren

Die Herstellung der erfindungsgemäßen Zubereitungen kann durch Vermischen der einzelnen Komponenten vorzugsweise bei Temperaturen oberhalb deren Schmelzpunkte erfolgen. Es ist jedoch ebenfalls möglich, die Quaternierung der den Esterquats zugrundeliegenden Alkanolaminfettsäureester in Gegenwart der Ölkörper und/oder Emulgatoren durchzuführen. Auf diesem Wege werden unmittelbar lösemittelfreie Produkte erhalten, die sich leicht zu Schuppen verarbeiten lassen.

### Gewerbliche Anwendbarkeit

Die erfindungsgemäßen Zubereitungen lösen sich spontan unter Emulsionsbildung in Wasser und ergeben dann schon bei sehr geringen Einsatzmengen von 1 bis 10 und vorzugsweise 2 bis 5 Gew.-% auch bei längerer Lagerung und Temperaturbelastung stabile Emulsionen, die unmittelbar für kosmetische Zwecke, beispielsweise als Haarkonditioniermittel verwendet werden können. Ein weiterer Gegenstand der Erfindung betrifft daher die Verwendung der genannten Zubereitungen zur Herstellung von kosmetischen Mitteln.

### Kosmetische Mittel

Die kosmetischen Mittel, wie beispielsweise Haarshampoos, Haarkonditioniermittel, Haarlotionen, Schaumbäder, Cremes oder Lotionen, können ferner als weitere Hilfs- und Zusatzstoffe milde Tenside, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen; sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, Ionon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkom-mission der Deutschen Forschungsgemeinschaft, Verlag Chemie, Weinheim, 1984, S.81-106** zu-sammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die in der nachstellenden Tabelle 1 dargestellten Zubereitungen wurden 4 Gew.-%ig in Wasser (16°D, 20°C) dispergiert. Die Dispergierbarkeit wurde nach folgender Skala bewertet: (+) = mehrmaliges Umrühren erforderlich, (++) = leichtes Umrühren erforderlich, (+++) = spontane Emulsionsbildung. Die Viskosität wurde nach der Brookfield-Methode (RVT-Viskosimeter, Spindel 1, 20°C, 10 Upm) nach 24 h und 7 d Lagerung beurteilt. Die Homogenität der Lösungen wurde nach einer Lagerung von 7d visuell nach folgender Skala eingestuft: (-) = Phasentrennung, (+) = leichte Trübung, (++) = keine Trübung. Die Beispiele 1 bis 4 sind erfindungsgemäß, die Beispiele V1 bis V4 dienen zum Vergleich.

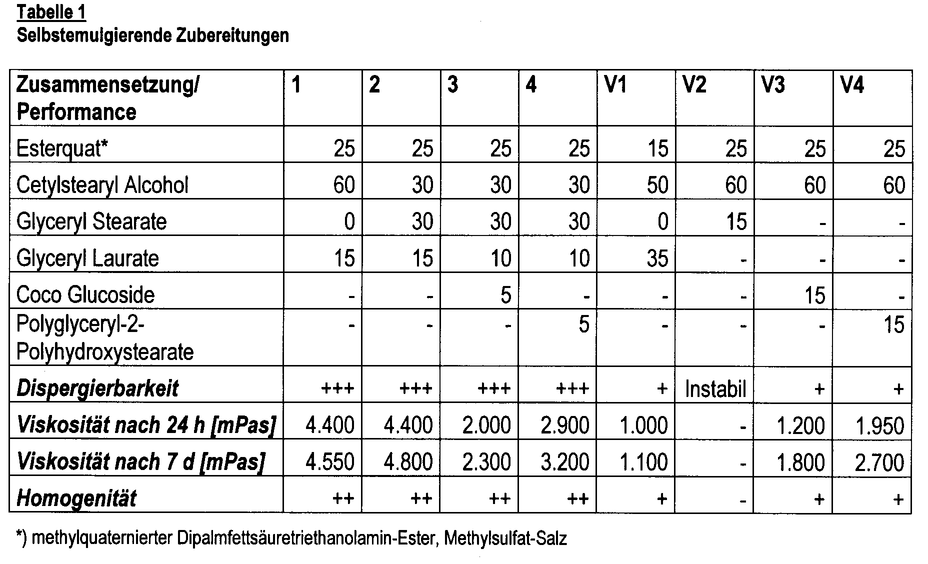

## Patentansprüche

1. Selbstemulgierende Zubereitungen, enthaltend
(a) 20 bis 25 Gew.-% Esterquats
(b) 60 bis 65 Gew.-% Ölkörper und
(c) 10 bis 20 Gew.-% Emulgatoren vom Typ der Partialglyceride von Fettsäuren mit 6 bis 14 Kohlenstoffatomen,
mit der Maßgabe, daß sich die Mengenangaben zu 100 Gew.-% ergänzen.

2. Zubereitungen nach Anspruch 1, **dadurch gekennzeichnet**, daß sie Esterquats der Formel **(I)** enthalten, 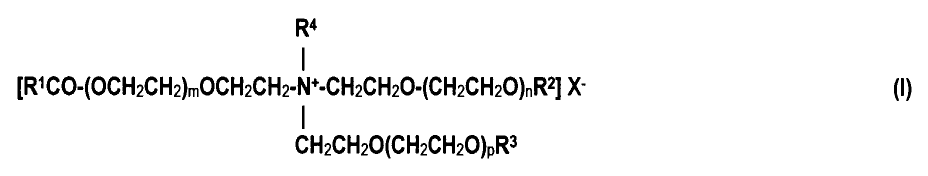 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Zubereitungen nach Anspruch 1, **dadurch gekennzeichnet**, daß sie Esterquats der Formel **(II)** enthalten, 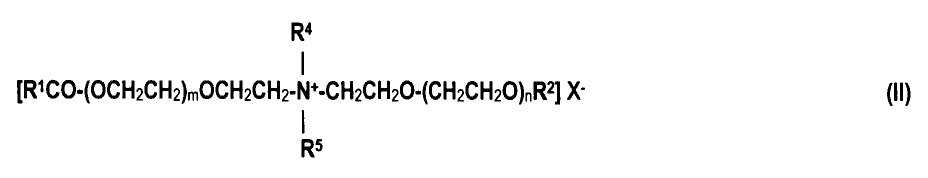 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Zubereitungen nach Anspruch 1, **dadurch gekennzeichnet**, daß sie Esterquats der Formel **(III)** enthalten, 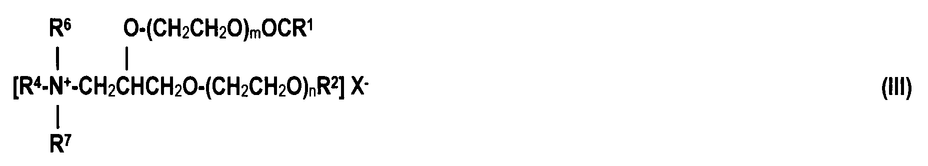 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Zubereitungen nach den Ansprüchen 1 bis 4, **dadurch gekennzeichnet**, daß sie als Ölkörper Fettalkohole der Formel **(IV)** enthalten, 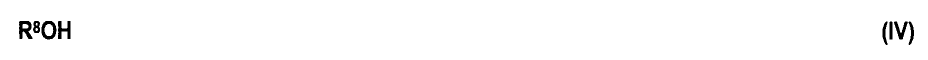 in der R⁸ für einen lienaren oder verzweigten Alkyl- und/oder Alkenylrest mit 6 bis 22 Kohlenstoffatomen steht.

6. Zubereitungen nach den Ansprüchen 1 bis 5, **dadurch gekennzeichnet**, daß sie als Ölkörper Partialglyceride der Formel **(V)** enthalten, 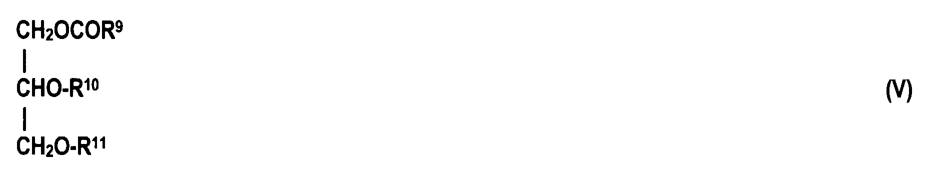 In der R⁹CO für einen linearen oder verzweigten, gesättigten und/oder ungesättigten Acylrest mit 16 bis 22 Kohlenstoffatornen, R¹⁰ und R¹¹ unabhängig voneinander für Wasserstoff oder R⁹CO steht, mit der Maßgabe, daß mindestens einer der beiden Reste R¹⁰ und R¹¹ Wasserstoff darstellt.

7. Zubereitungen nach den Ansprüchen 1 bis 6, **dadurch gekennzeichnet**, daß sie als Emulgatoren Partialglyceride der Formel **(VI)** enthalten, 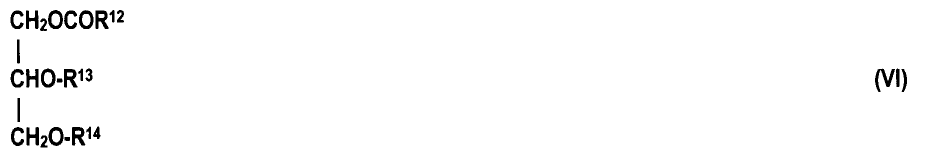 in der R¹²CO für einen Acylrest mit 12 bis 14 Kohlenstoffatomen, R¹³ und R¹⁴ unabhängig voneinander für Wasserstoff oder R¹²CO steht, mit der Maßgabe, daß mindestens einer der beiden Reste R¹³ und R¹⁴ Wasserstoff darstellt.

8. Zubereitungen nach den Ansprüchen 1 bis 7, **dadurch gekennzeichnet**, daß sie als weitere Emulgatoren Alkyl- und/oder Alkenyloligoglykoside enthalten.

9. Zubereitungen nach den Ansprüchen 1 bis 8, **dadurch gekennzeichnet**, daß sie als weitere Emulgatoren Polyolpoly-12-hydroxystearate enthalten.

10. Verwendung von Zubereitungen nach den Ansprüchen 1 bis 9 zur Herstellung von kosmetischen Mitteln.
